# EUROPEAN PATENT APPLICATION

(11) **EP 3 311 735 A1**
(43) Date of publication of application: **25.04.2018**
(21) Application number: 16194597.7
(22) Date of filing: 19.10.2016
(51) Int. Cl.: A61B 5/01, A61B 5/024

(54) **FLEXIBLE APPARATUS**

(71) Applicant: King's Metal Fiber Technologies Co., Ltd., 42060 Taichung City (TW)
(72) Inventor: CHEN, Reng Sho, 10451 Taipei City (TW); LIAO, Shu Fen, 10451 Taipei City (TW); KANG, Yu Hsun, 10451 Taipei City (TW)
(74) Representative: Cabinet Chaillot

(57) **Abstract**

A flexible device includes two devices (10) and a flexible interface (20). The two devices (10) each include electrical components (11) assembled therein. The flexible interface (20) is coupled to the two devices (10) such that the flexible interface (20) connects the two devices (10) to each other. When the two devices (10) are positionable on a human body, the flexible interface provides stretchability and extendability so that the two devices (10) can be set on two opposite sides of a protrusion site of bones of the human body, allowing for stretching and contracting of the human body to provide an effect of suiting the ergonomic needs.

## Description

### BACKGROUND OF THE INVENTION

### 1. Field of the Invention

The present invention relates generally to a flexible apparatus, and more particularly to a combined arrangement of at least two devices and a flexible interface, where the flexible interface is stretchable or extendible to allow the at least two devices to be positionable on two opposite sides of a protrusion site of human skeleton for easy stretching and contraction of the human skeleton, thereby being applicable to serving as physiological detection instruments, medical inspection apparatus, and the likes.

### 2. Description of Related Art

Contemporary physiological detection instruments or medical inspection apparatus are generally structured such that a stiff housing or enclosure is involved for protection of internally-arranged electronic components/devices of such instruments or apparatus against impact and preventing undesired influences caused thereby.

Human bodies are generally formed of around 206 bones and muscles and a surface skin formed outside and surrounding and housing the bones. Movements that a human body may take may cause some bones to shape up on the skin. The human bones are generally a rigid organ that supports and protects the human body.

When a human body respires, the chest may move up and down and the skin is caused to stretch and contract. A physiologic detection instrument or a medical inspection apparatus, when positioned on the skin surface of a human body, would make the stiff enclosure of the physiological detection instrument or the medical inspection device rubbing the skin surface of the human body and thus causing discomfort of the human body and even discouraging use of such apparatus or instruments.

Further, although some physiological detection instruments or medical inspection devices are claimed to be of a design for fitting to human body contour, the contours of human bodies are generally not consistent due to factors of for example heights and growing conditions. In addition, the physiological detection instruments or medical inspection devices are generally mounted to the skin surface at a site where the bones shape up, and therefore, when a physiological detection instrument or a medical inspection device is attached to such a bone shape-up site, it generally causes discomfort and inconvenience to some extents and may readily cause incorrect readings of detection or inspection or discontinuity of detection or inspection signals. This is additional suffering for the use.

Thus, in view of the above problems, for the purpose of providing a flexible apparatus that meets the need for certain desired performance and effectiveness by allowing for easy operation and installation by users, the present inventor has been devoted himself to study and design in order to provide such convenience of use for the users. This is the motivation of creation of the present invention.

### SUMMARY OF THE INVENTION

The primary object of the present invention is to provide a flexible device, which comprises a combination of at least two devices and a flexible interface, wherein the at least two devices each comprise electrical components assembled therein and the flexible interface are coupled to the at least two devices such that the flexible interface connects the at least two devices to each other in such a way that when the at least two devices are positioned on a human body, stretchability and extendability is achieved with the flexible interface, allowing the at least two devices to be set on opposite sides of a protrusion site of human bones for easy stretching and contraction of the human bones so as to suit ergonomic needs of the user thereby improving overall utilization.

Another object of the present invention is to provide a flexible device, wherein the flexible interface is arranged between the at least two devices and the flexible interface has two ends respectively connected to the at least two devices, in which the connection is achieved with one of ultrasonic fusion, insert molding, adhesive bonding, and mating engagement between corresponding projection and recess, to allow the at least two devices can be installed to adapt to human portions of joints, spine bones, chest bones, wrist bones, elbows, and knee joints of human body by means of the characteristics of the flexible interface arranged therebetween that allows for bending or forming a curved surface, to prevent the feeling of discomfort of the human body and thus improving overall utilization.

Another object of the present invention is to provide a flexible device, wherein the flexible interface comprises at least two cavities formed therein to respectively corresponding to the at least two devices and the at least two devices are respectively received in and assembled within the cavities, in which the cavities are provided therein with electrical contact terminals and the devices are also provided with electrical contact terminals, so that when the devices are assembled with in the cavities of the flexible interface, conductive engagement can be formed through the electrical contact terminals of the devices and 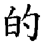 the electrical contact terminals of the cavities of the flexible interface to allow the at least two devices to take deformation of the flexible interface arrange therebelow through the flexibility thereof to adapt to human portions of joints, spine bones, chest bones, wrist bones, elbows, and knee joints of human body and also to allow for reduction of discomfort of human body through the flexible interface thereby improving overall utilization.

Another object of the present invention is to provide a flexible device, wherein the at least two devices are each divided into an upper portion and a lower portion and the upper portion and the lower portion are each provided with electrical contact terminals, while the flexible interface is arranged between the upper portion and the lower portion of each of the at least two devices and the flexible interface is provided with electrical contact terminals, so that when the upper portions and the lower portions of the devices are combined with each other, the electrical contact terminals of the upper portions and the lower portions of the devices form conductive engagement with the electrical contact terminals of the flexible interface arranged therebetween and the upper portions and the lower portions of the devices may change angular positions thereof through the flexible interface, providing the devices with an effect of stretchability for being positionable on any portion of a human body without being interfered with by a protrusion site of the human body thereby improving overall utilization.

A further object of the present invention is to provide a flexible device, in which the devices are connected with at least one signal line and the signal line has an opposite end that is connected to a detection element, wherein the detection element is configured as an electrode plate that is capable of detecting a physiological signal on a surface of a human body and the physiological signal is one of body temperature, heartbeat, and pulse, so that the devices receive and collect the physiological signal detected by the detection element and recording and storage can be made with the devices and the detected physiological signal can be transmitted, in a wireless manner, to a smart handheld electronic device or an electronic instrument that is capable of receiving the physiological signal for achieving an effect of analysis and management of physiological condition thereby improving overall utilization.

To achieve the above objects, the present invention provides a flexible device, which comprises at least two devices and a flexible interface. The at least two devices each comprise electrical components assembled therein and the flexible interface is coupled to the at least two devices such that the flexible interface connects the at least two devices to each other, whereby the at least two devices are positionable on a human body with stretchability and extendibility achieved with the flexible interface.

The above and other objects and advantages of the present invention can be understood from a detailed description given below with reference to preferred embodiments of the present invention and the attached drawings.

It is apparent that some of the components or arrangement of components of the present invention may be varied or modified, yet the embodiments illustrated are provided with a detailed description in the specification and structures thereof are illustrated in the attached drawings.

### BRIEF DESCRIPTION OF THE DRAWINGS

FIG. 1 is a perspective view showing a primary embodiment of the present invention;
FIG. 2 is a perspective view showing a channel of a flexible interface of the primary embodiment of the present invention;
FIG. 3 is a perspective view illustrating, in an assembled form, the flexible interface of the primary embodiment of the present invention comprising a projection section or a device comprising a recessed section for jointing engagement;
FIG. 4 is a perspective view illustrating, in an exploded form, the flexible interface of the primary embodiment of the present invention comprising a projection section or a device comprising a recessed section for jointing engagement;
FIG. 5 is a perspective view showing another embodiment of the present invention;
FIG. 6 is an exploded view showing said another embodiment of the present invention;
FIG. 7 is a perspective view showing a further embodiment of the present invention;
FIG. 8 is an exploded view showing said further embodiment of the present invention; and
FIG. 9 is a schematic view showing a use of the present invention.

### DETAILED DESCRIPTION OF THE PREFERRED EMBODIMENTS

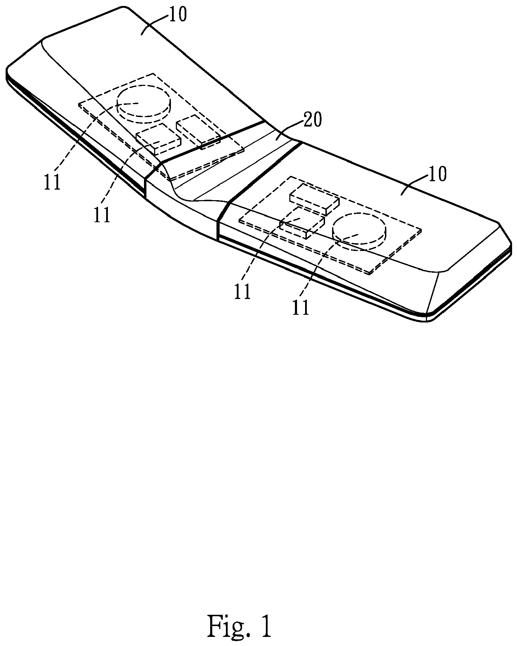

Referring to FIGS. 1-9, drawings are provided to illustrate embodiments of the present invention. The present invention provides a flexible device of which a preferred embodiment is applicable to a surface of a human body to prevent undesired interference with physiological detection caused by protrusion of human bones and is generally fit to installation on parts of the human body including joints, spine bones, chest bones, wrist bones, elbows, and knee joints of the human body in order to suit the needs of different persons without causing undesired feeling of discomfort of human body.

A primary embodiment of the flexible device of the present invention comprises at least two devices 10 and a flexible interface 20. The at least two devices 10 are each assembled with electrical components 11, and the flexible interface 20 is coupled to the at least two devices 10 (as shown in FIG. 1) such that the flexible interface 20 connects the at least two devices 10 to each other in such a way that when the at least two devices 10 are positioned on a human body, stretching or extension can be achieved with the flexible interface 20.

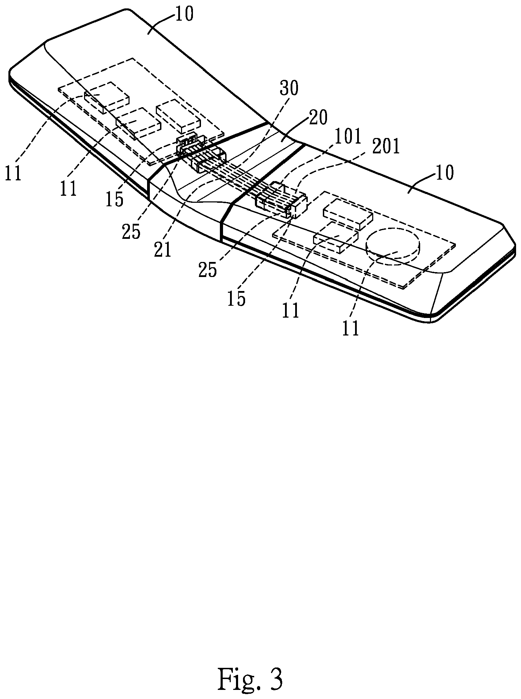

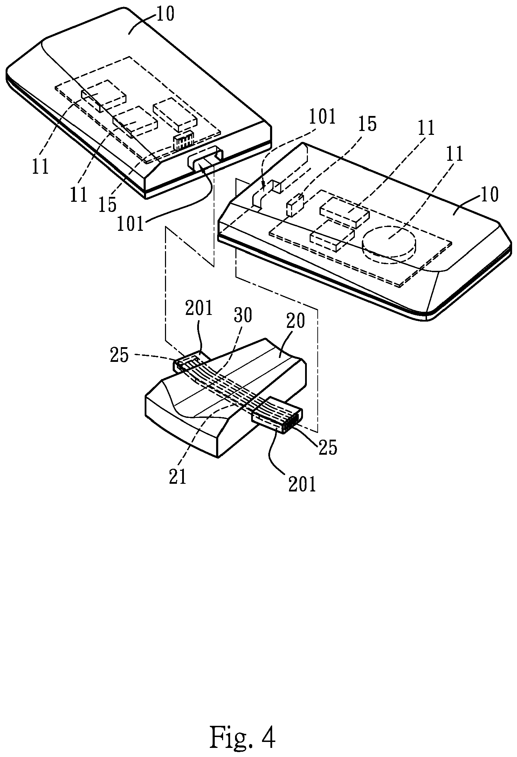

In the primary embodiment of the present invention, the flexible interface 20 is assembled between the at least two devices 10 and the flexible interface 20 has two opposite ends that are respectively connected to the at least two devices 10, wherein the connection between the two ends of the flexible interface 20 and the at least two devices 10 can be achieved with one of ultrasonic fusion, insert molding, adhesive bonding, mating engagement between corresponding projection and recess, and is exemplified, in the embodiment illustrated in the drawings, in the form of mating engagement between corresponding projection and recess (but not limited to what shown in the drawings). The at least two devices 10 are each provided with a recessed section 101 in opposing sides thereof and the two ends of the flexible interface 20 are each extended to form a projecting section 201, such that the projecting sections 201 of the two ends of the flexible interface 20 are receivable into and engageable with and thus coupled to the recessed sections 101 of the at least two devices 10 (as shown in FIGS. 3 and 4), whereby the at least two devices 10 can positioned on body portions of joints, spine bones, chest bones, wrist bones, elbows, and knee joints of human body by using the characteristics of the intermediately connecting flexible interface 20 that may be set in a bending form or showing a curved surface to comply with the human body portions and thus prevent discomfort feeling of the human body.

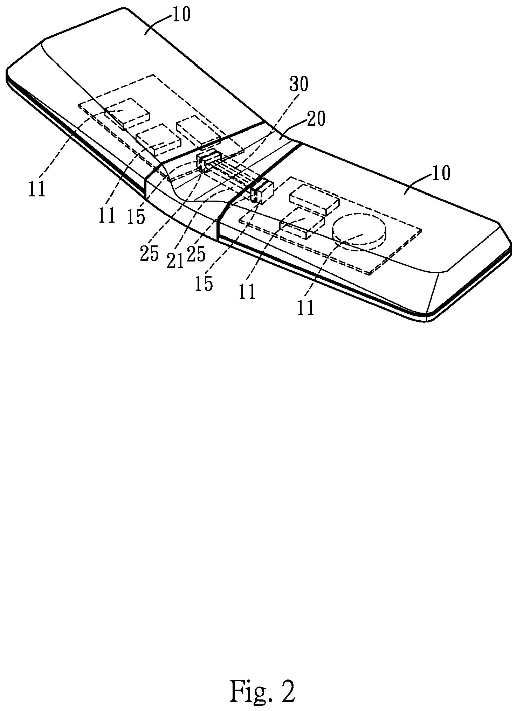

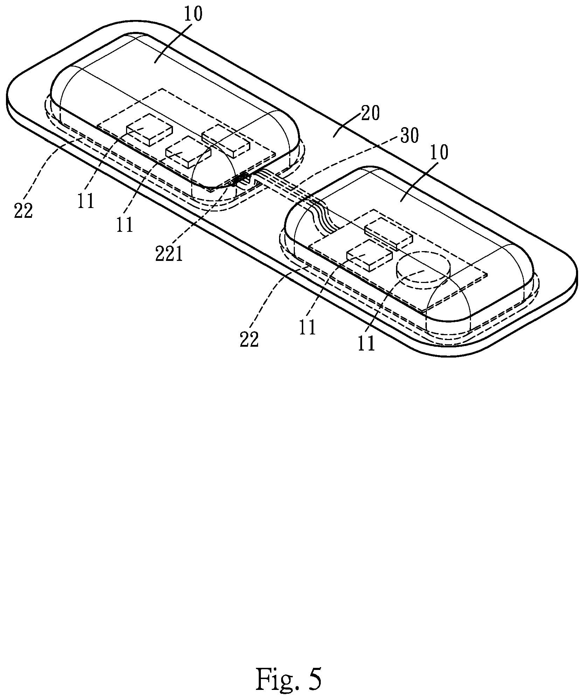

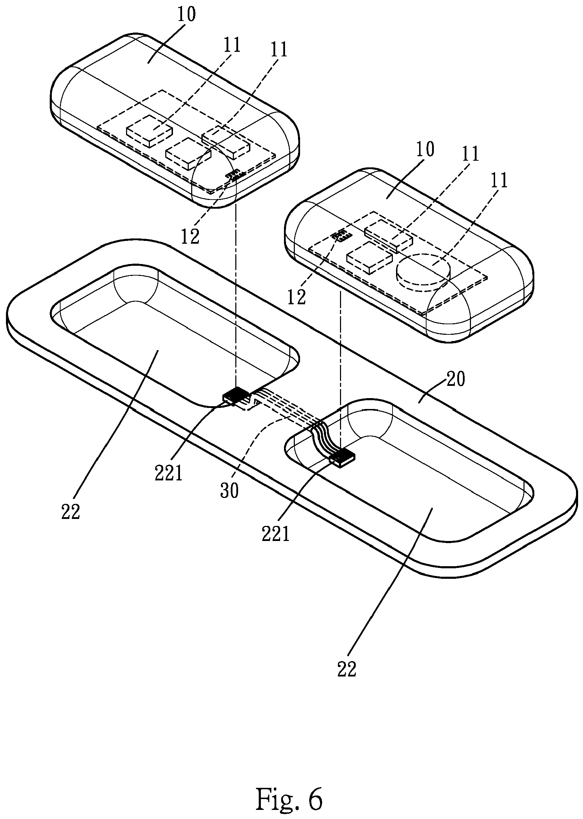

In addition, the flexible interface 20 may be arranged to have two different structures, one being that the flexible interface 20 has a structure that is completely solid with no arrangement of hole or opening formed in the flexible interface 20 in order to separate the at least two devices 10 completely from each other (as shown in FIG. 1), whereby no direct connection between the at least two devices 10 is possible for being independent of each other. The second form of the structure of the flexible interface 20 is that the flexible interface 20 is provided, in the interior thereof, with a channel 21 (as shown in FIG. 2) to allow the at least two devices 10 to be assembled with at least one transmission line 30 through the channel 21 of the flexible interface 20, and the flexible interface 20 is provided therein with electrical contact terminals 25 and the devices 10 are also provided with electrical contact terminals 15, such that when the devices 10 and the flexible interface 20 are assembled together, the electrical contact terminals 15 of the devices 10 may form conductive engagement with the electrical contact terminals 25 of the flexible interface 20 (as shown in FIGS. 3 and 4), and electrical connection can be formed between the at least two devices 10 through the at least one transmission line 30 so that communication can be achieved through transmission of signal between the at least two devices 10. Besides what discussed above, the connection that can be adopted in the present invention may include direct insert molding of the transmission line 30 inside the flexible interface 20 (not illustrated, but reference being made to the illustrations of FIGS. 5 and 6), so that communication can be achieved through transmission of signal between the at least two devices 10.

The above-described devices 10 can be one or two of signal transceivers, power supply devices, wireless signal transceivers, detection modules, GPS (Global Positioning System) modules, incline sensor modules, control processing modules, and coupling devices with electrical property, and the electrical components 11 can be one or a combination of electrical components including incline sensor chips, microcontrollers, wireless transmission chips, power control chips, signal transmission chips, electrical cells/batteries. In addition, the flexible interface 20 can be one of rubber, silicone rubber, plastics, and woven fabric.

Another embodiment of the flexible device of the present invention is similar to the primary embodiment in that at least two devices 10 and a flexible interface 20 are included, where the devices 10, the electronic components 11, and the flexible interface 20 are similar, in contents and assembly thereof, to those of the primary embodiment so that reference is made to the above description and repeated description will be omitted here. The following is made to illustrate differences between said another embodiment and the primary embodiment.

Said another embodiment of the present invention is such that the flexible interface 20 is provided with at least two cavities 22 formed therein to respectively correspond to the at least two devices 10. The flexible interface 20 has a surface area that is much greater than surface areas of the at least two devices 10. The at least two devices 10 are respectively received in and assembled within the cavities 22, wherein the cavities 22 are such that the contours or shapes of the cavities 22 are displayed on a surface of the flexible interface 20 (as shown in FIG. 6), or alternatively, the surface layer of the flexible interface 20 is thickened such that the cavities 22 are located inside the surface layer of the flexible interface 20 without exhibiting the shapes or contours of the cavities 22 on the surface of the flexible interface 20.

Electrical contact terminals 221 are provided inside the cavities 22 of the flexible interface 20 and the devices 10 that are corresponding to and respectively assembled within the cavities 22 are also provided with electrical contact terminals 12, such that the devices 10, when assembled within the cavities 22 of the flexible interface 20, may form conductive engagement with 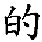 the electrical contact terminals 221 of the cavities 22 of the flexible interface 20 through the electrical contact terminals 12 of the devices 10. Further, the flexible interface 20 is provided therein with a channel (not shown in the drawings but reference being had to the illustrations of FIGS. 3 and 4) between the at least two cavities 22 to allow the at least two cavities 22 to connect with at least one transmission line 30 through the channel for formation electrical connection between the at least two cavities 22. In the drawings of said another embodiment of the present invention, the transmission line 30 is exemplified as being formed in the flexible interface 20 by means of direct insert molding (as shown in FIGS. 5 and 6), so that communication can be achieved through transmission of signal between the at least two devices 10 and the at least two devices 10 can adapt to human portions such as joints, spine bones, chest bones, wrist bones, elbows, and knee joints of human body through deformation of the flexible interface 20 through flexibility thereof and discomfort feeling of the human body can be reduced by means of the flexible interface 20.

A further embodiment of the flexible device of the present invention is similar to the primary embodiment in that at least two devices 10 and a flexible interface 20 are included, where the devices 10, the electronic components 11, and the flexible interface 20 are similar, in contents and assembly thereof, to those of the primary embodiment so that reference is made to the above description and repeated description will be omitted here. The following is made to illustrate differences between said further embodiment and the primary embodiment.

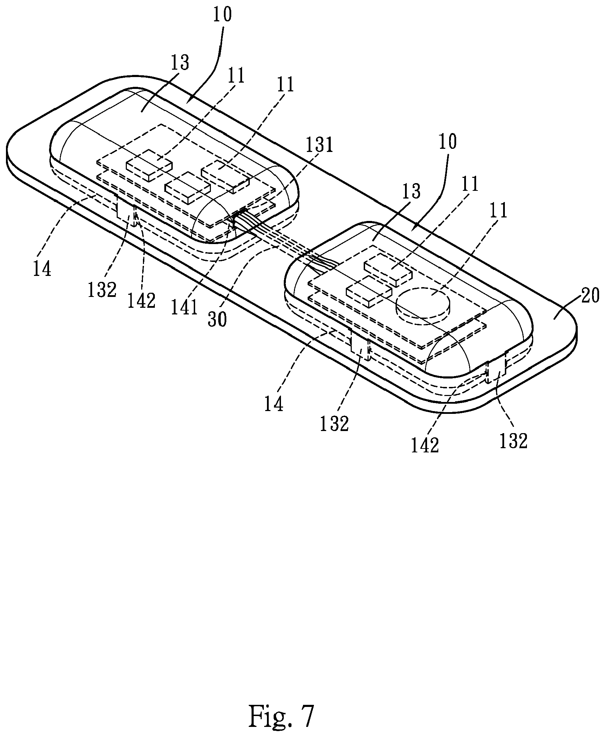

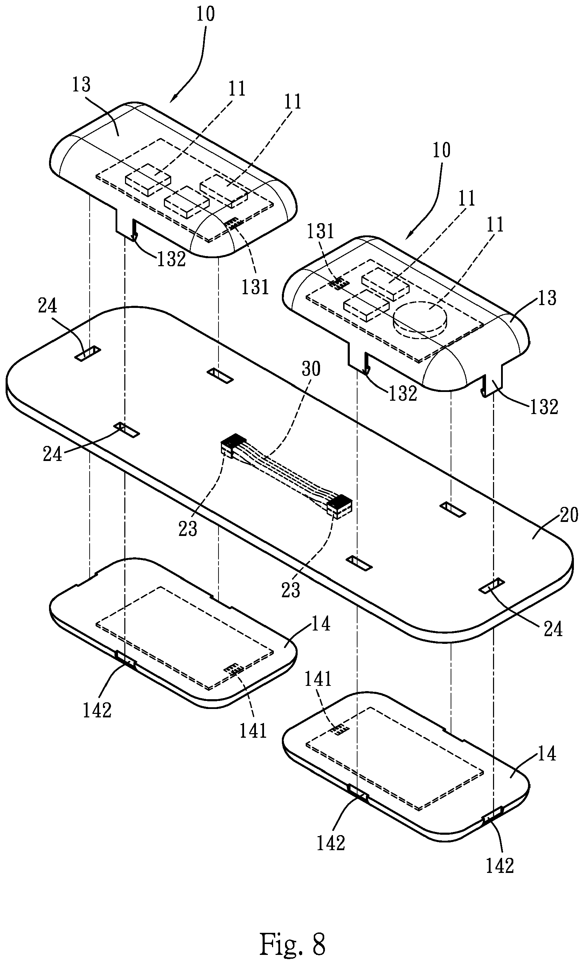

Said further embodiment of the present invention is such that the at least two devices 10 are each divided into an upper portion 13 and a lower portion 14, and the upper portion 13 and the lower portion 14 are respectively provided with electrical contact terminals 131, 141. The flexible interface 20 is arranged between the upper portion 13 and the lower portion 14 of each of the at least two devices 10 (as shown in FIG. 7 and 8), and the flexible interface 20 is provided thereon with electrical contact terminals 23 at locations corresponding to the electrical contact terminals 131, 141 of the upper portion 13 and the lower portion 14. When the upper portions 13 and the lower portions 14 of the devices 10 are combined together, the electrical contact terminals 131, 141 of the upper portions 13 and the lower portions 14 of the devices 10 may for conductive engagement with the electrical contact terminals 23 of the flexible interface 20 so that he upper portions 13 and the lower portions 14 of the at least two devices 10 may change directions or angular positions thereof by means of the flexible interface 20 and an effect of extendability or stretchability may be provided to the at least two devices 10, allowing them to be positionable on any part of a human body without being interfered with by any projecting portion of the human body.

The flexible interface 20 is provided with holes 24, and the upper portion 13 and the lower portion 14 of each of the devices 10 are respectively provided with retention engagement sections 132, 142 corresponding thereto. With the retention engagement sections 132, 142 of the upper portion 13 or the lower portion 14 of each of the devices extending through the corresponding holes 24 of the flexible interface 20 (as shown in FIG. 8) to mate and engage the retention engagement sections 132, 142 of the upper portion 13 or the lower portion 14 of the device 10 for coupling together, the upper portion 13 and the lower portion 14 of each of the devices 10 can be securely coupled to the flexible interface 20. (The mating retention engagement can be of alternative and different designs and is not limited to what illustrated in the drawings.) As such, the electrical contact terminals 131, 141 of the upper portion 13 and the lower portion 14 of each of the devices 10 can be set in conductive engagement with the electrical contact terminals 23 of the intermediately arranged flexible interface 20. The flexible interface 20 is provided therein with at least one transmission line 30, such that the transmission line 30 provides electrical connection between the electrical contact terminals 23 of the flexible interface 20 to allow the at least two devices 10 to transmit signals therebetween for communication or connection.

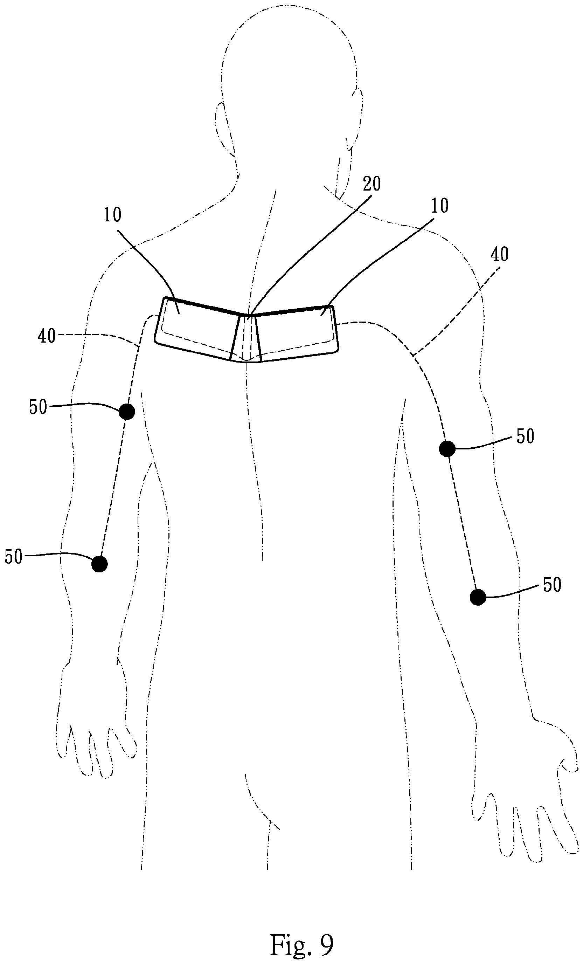

Finally, each of the primary embodiment, said another embodiment, and said further embodiment of the present invention allows the devices 10 to be provided with at least one signal line 40. An oppose end of the signal line 40 is provided with a detection element 50 (as shown in FIG. 9), and the detection element 50 comprises electrode plate for detecting a physiological signal on a surface of a human body. The physiological signal can be one of body temperature, heartbeat, and pulse, so that the devices 10 may receive and collect the physiological signal detected by the detection element and recording and storage can be made with the devices 10 and the physiological signal so detected can be transmitted, in a wireless manner, to a smart handheld electronic device or an electronic instrument that is capable of receiving the physiological signal for achieving an effect of analysis and management of physiological condition.

Through the detailed description provided above, a person skilled in the art would appreciate that the purpose of the present invention can surely be realized with the technical solution of the present invention and thus, the present invention generally meet all postulated requirement of patent laws and regulations. And, as such a patent application is thus filed with the invention.

However, it is appreciated that the above description provides just preferred embodiments of the present invention and the description is not intended to impose constraint to the scope of the present invention that protection is sought through the appended claims. Thus, all the variations and modifications that are believed equivalent to the present invention according to wheat described and defined in the above specification and the appended claims should be regarded within the scope of protection covered by the present invention.

## Claims

1. A flexible device, comprising:
at least two devices (10), which are each assembled with electrical components (11); and
a flexible interface (20), which is coupled to the at least two devices (10) so that the flexible interface (20) connects the at least two devices (10) to each other, wherein the at least two devices (10) are positionable on a human body with stretchability achievable with the flexible interface (20).

2. The flexible device as claimed in Claim 1, wherein the flexible interface (20) is assembled between the at least two devices (10) and the flexible interface (20) has two opposite ends that are respectively connected to the at least two devices (10), the flexible interface (20) comprising a channel (21) formed in an interior thereof to allow the at least two devices (10) to be assembled with at least one transmission line (30) through the channel (21) of the flexible interface (20) so that the at least two devices (10) are electrically connected to each other.

3. The flexible device as claimed in Claim 2, wherein the connection of the two ends of the flexible interface (20) with the at least two devices (10) includes one of ultrasonic fusion, insert molding, adhesive bonding, and mating engagement between corresponding projection and recess.

4. The flexible device as claimed in Claim 1, wherein the flexible interface (20) comprises at least two cavities (22) formed therein to respectively corresponding to the at least two devices (10), the at least two devices (10) being respectively received in and assembled within the cavities (22), the at least two cavities (22) being provided therebetween with a channel to allow the at least two cavities (22) to be assembled with at least one transmission line (30) through the channel so that the at least two cavities (22) are electrically connected to each other.

5. The flexible device as claimed in Claim 4, wherein the cavities (22) of the flexible interface (20) are respectively provided therein with electrical contact terminals (221) and the devices (10) are provided with electrical contact terminals (12), such that the devices (10) assembled within the cavities (22) of the flexible interface (20) form conductive engagement with the electrical contact terminals (221) of the cavities (22) of the flexible interface (20) through the electrical contact terminals (12) of the devices (10).

6. The flexible device as claimed in Claim 1, wherein the at least two devices (10) each comprise an upper portion (13) and a lower portion (14), the upper portion (13) and the lower portion (14) being respectively provided with electrical contact terminals (131, 141), the flexible interface (20) being arranged between the upper portion (13) and the lower portion (14) of each of the at least two devices (10), the flexible interface (20) being provided with electrical contact terminals (23), the flexible interface (20) comprising at least one transmission line (30) arranged therein, such that the transmission line (30) forms electrical connection between the electrical contact terminals (23) of the flexible interface (20), wherein the upper portion (13) and the lower portion (14) of each of the devices (10) are combinable with each other such that the electrical contact terminals (131, 141) of the upper portion (13) and the lower portion (14) of each of the devices (10) form conductive engagement with the electrical contact terminals (23) of the flexible interface (20) that is arranged in between.

7. The flexible device as claimed in Claim 6, wherein the flexible interface (20) comprises holes (24) formed therein and the upper portions (13) and the lower portions (14) of the devices (10) are respectively provided with retention engagement sections (132, 142) mateable and engageable with each other so that the retention engagement sections (132, 142) of one the upper portion (13) and the lower portion (14) of each of the devices (10) are extendable through the holes (24) of the flexible interface (20) to mate and engage with and thus be coupled to the retention engagement sections (132, 142) of another one of the upper portion (13) and the lower portion (14) of the device (10).

8. The flexible device as claimed in Claim 1, 2, 4, or 6, wherein the devices (10) is connected with at least one signal line (40), the signal line (40) having an opposite end to which a detection element (50) is connected, the detection element (50) being configured as an electrode plate for detecting a physiological signal on a surface of a human body, the physiological signal being one of body temperature, heartbeat, and pulse.

9. The flexible device as claimed in Claim 1, wherein the at least two devices (10) comprise one or two of signal transceivers, power supply devices, wireless signal transceivers, detection modules, global positioning system (GPS) modules, incline sensor modules, control processing modules, and coupling devices with electrical property.

10. The flexible device as claimed in Claim 1, wherein the electrical components (11) comprise one or a combination of electrical components including incline sensor chips, microcontrollers, wireless transmission chips, power control chips, signal transmission chips, and electrical cells/batteries.

11. The flexible device as claimed in Claim 1, wherein the flexible interface (20) comprises one of rubber, silicone rubber, plastics, and woven fabric.
